# EUROPEAN PATENT SPECIFICATION

(11) **EP 2 522 323 B1**
(45) Date of publication and mention of the grant of the patent: **28.06.2017**
(21) Application number: 11731804.8
(22) Date of filing: 05.01.2011
(51) Int. Cl.: A61F 13/15, A61F 13/49, A61F 13/494, A61F 13/72, A61F 13/496

(54) **DISPOSABLE WEARING ARTICLE**
EINWEG-GEBRAUCHSARTIKEL
ARTICLE À PORTER JETABLE

(30) Priority: 08.01.2010 JP 2010003414
(43) Date of publication of application: 14.11.2012
(73) Proprietor: Unicharm Corporation, Ehime 799-0111 (JP)
(72) Inventor: SASAYAMA, Kenichi, Kanonji-shi Kagawa 769-1602 (JP); ICHIKAWA, Makoto, Kanonji-shi Kagawa 769-1602 (JP); YAMAMOTO, Hiroki, Kanonji-shi Kagawa 769-1602 (JP); UKEGAWA, Kazuo, Kanonji-shi Kagawa 769-1602 (JP); NINOMIYA, Akihide, Kanonji-shi Kagawa 769-1602 (JP)
(74) Representative: Peter, Julian
(86) International application number: PCT/JP2011/050062
(87) International publication number: WO 2011/083806

(56) References cited:
- WO-A2-02/13748
- JP-A- 2000 271 167
- JP-A- 2001 269 368
- JP-A- 2003 010 237
- JP-A- 2004 329 590
- JP-A- 2004 505 726

## Description

### {Technical Field}

The present invention relates to disposable wearing articles and more particularly to disposable wearing articles such as disposable diapers, disposable toilet-training pants, disposable incontinent pants, disposable sanitary pants and the like, each provided in a crotch region thereof with leg elastic elements secured thereto in a curved state

### {background}

Conventionally, wearing articles including elasticized lateral portions adapted to be put in close contact with the wearer's thighs are known. For example, JP 2000-254176 A (PTL 1) discloses a disposable wearing article including a pair of elasticized lateral portions on both sides of a liquid-absorbent structure each provided with a plurality of strand- or string-like leg elastic elements.

### {Citation List}

### {Patent Literature}

{PTL 1} JP 2000-254176 A

JP 2000 271167 A discloses an absorbent article such as an open-type disposable diaper provided with tape fasteners to fasten front and rear waist regions, which includes a plurality of first leg elastic elements extending rectilinearly in the longitudinal direction inboard of the side edges of the crotch region, and a plurality of second leg elastic elements extending in the longitudinal direction outboard of the first leg elastic elements wherein the second leg elastic elements have shapes concavely curved in the middle zone of the crotch region and in the vicinity thereof. This patent literature is silent about a correlation between tensile stresses on the first and second leg elastic elements.

JP 2004 505726 A discloses an absorbent garment such as open-type disposable diaper provided with tape fasteners to fasten front and rear waist regions, which includes a plurality of first leg elastic elements extending rectilinearly in the longitudinal direction inboard of the side edges of the crotch region, and a plurality of second leg elastic elements extending rectilinearly in the longitudinal direction inboard of the first leg elastic elements, but longitudinal end portions of the second leg elastic elements are curved outward. This patent literature is silent about a correlation between the first and second leg elastic elements.

JP 2001 269368 A discloses an open-type disposable diaper provided with tape to fasteners fasten front and rear waist regions, which includes a plurality of leg elastic elements extending rectilinearly in the middle zone of the crotch region and curving outward in the longitudinal end portions thereof.

JP 2004 329590 A discloses a pant-type diaper, which includes a plurality of first and second leg elastic elements which are in parallel with each other in the longitudinal direction. This patent literature is silent about a correlation between tensile stresses of the first and second leg elastic elements.

### {Summary}

### {Technical Problem}

In wearing articles such as disposable diapers, it is generally required for a crotch region to have, in its middle and in the vicinity thereof, a fit better than that in the other regions in order to prevent body waste from sideway leaking out of the article. In the wearing article according to the invention disclosed in PTL 1, each of elasticized lateral portions is provided in its entire area in a transverse direction with leg elastic elements extending in a longitudinal direction in parallel one to another at regular intervals. With such an arrangement, it is impossible to make a tensile stress in the middle of the crotch region outstanding. While the tensile stress generated by the leg elastic elements may be locally enhanced by adjusting the total number or the total tensile stress of the leg elastic elements, when such measure is adopted that the elasticized lateral portions as a whole should excessively thighten the wearer's legs and create a feeling of discomfort against the wearer. Furthermore, to increase the number of the elastic elements locally, an additional step for this purpose will be required.

An object of the present invention is to provide a disposable wearing article improved so that the tensile stress in the middle of the crotch region as well as in the vicinity thereof can be set to be higher than in the other regions without adjusting the number and thickness of the leg elastic elements and a relatively large body waist retaining space can be formed to protect the wearer's skin from being soiled with body waste.

### {Solution to Problem}

According to the present invention, there is provided a disposable wearing article according to claim 1.

The present invention additionally includes embodiments as follows: The wearing article includes a liquid-absorbent structure attached to the side of the crotch region facing the wearer's skin and elasticized lateral portions adapted to be spaced upward from the liquid-absorbent structure under contraction of the leg elastic elements.

A dimension by which the second leg elastic elements are spaced from the first leg elastic elements is gradually reduced as the first and second leg elastic elements get closer to the middle zone of the crotch region.

The crotch member has front and rear ends and at least one of the front and rear ends is attached to the outer surface of the elasticized waist panel.

Each of the second leg elastic elements includes a concavely curved segment in ) the middle zone of the crotch region and in the vicinity thereof and a pair of rectilinear segments being contiguous to the concavely curved segment and respectively extending to ends of the second leg elastic elements opposite in the longitudinal direction wherein the concavely curved segment has a tensile stress higher than that of the rectilinear segment.

### {Advantageous Effects of Invention}

According to the present invention, the crotch region is provided along side edges thereof with a plurality of leg elastic elements contractibly attached thereto under tension so as to extend in the longitudinal direction. Of the elastic elements defining the leg elastic elements, the elastic elements lying on the outside as viewed in the transverse direction have concavely curved segments in the longitudinally middle zone of the crotch region and in the vicinity thereof. With this unique arrangement, the tensile stress of the regions defined by these concavely curved segments may be set to be higher than in the other regions without varying the kind, the number and the thickness of the elastic elements.

### {Brief Description of Drawings}

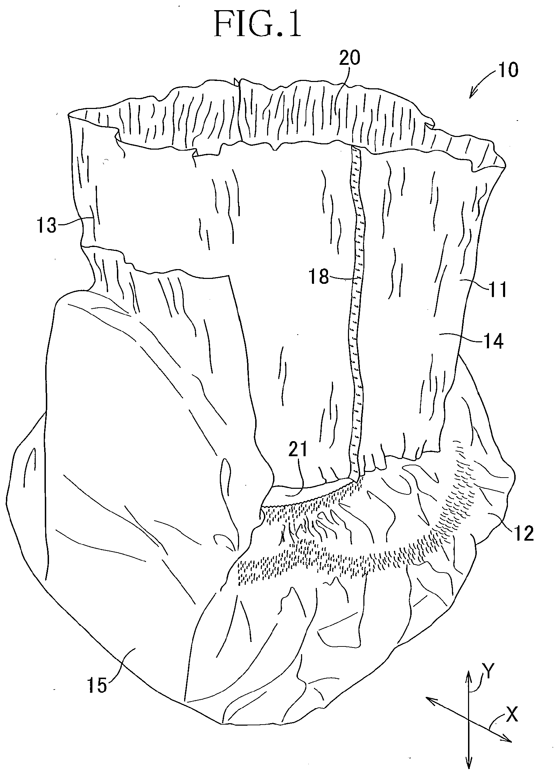

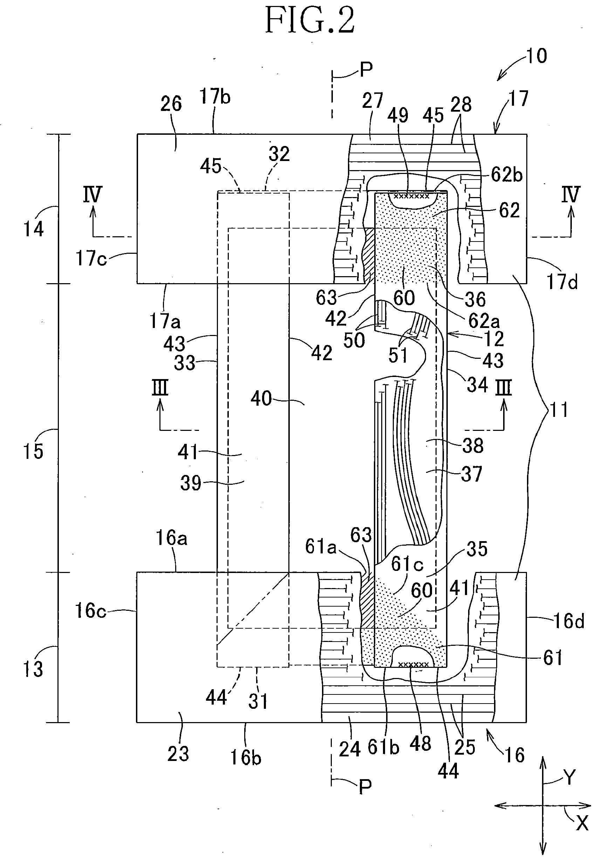

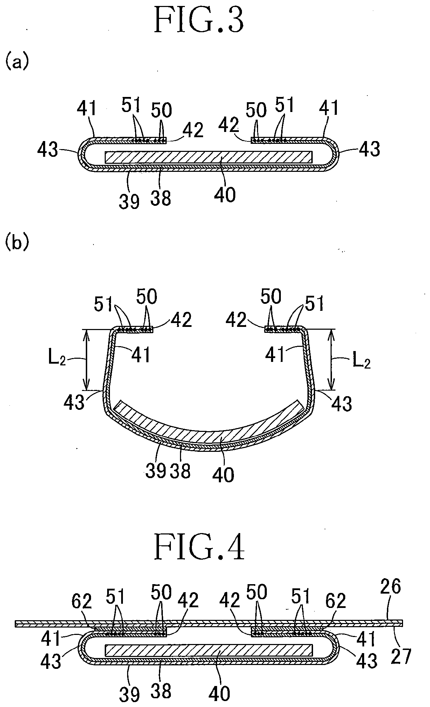

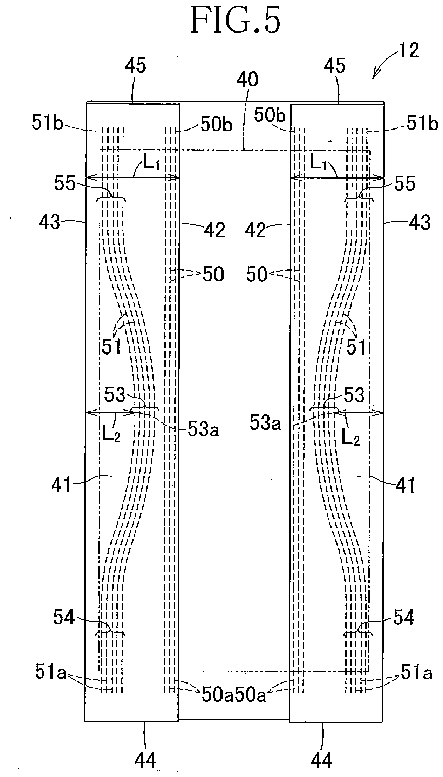

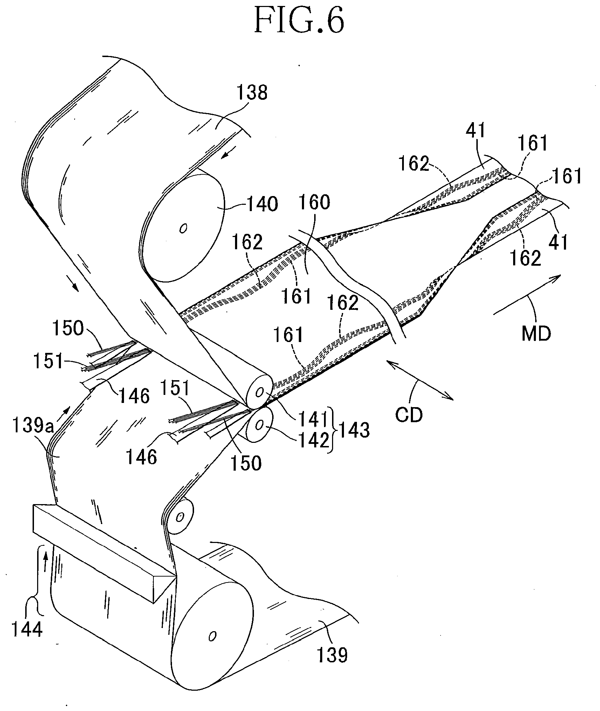

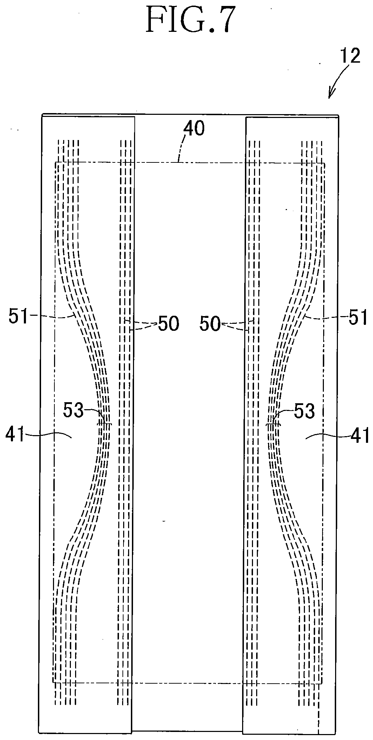

{Fig. 1} Fig. 1 is a perspective view showing a disposable diaper as one example of disposable wearing articles according to a first embodiment of the present invention.
{Fig. 2} Fig. 2 is a partially cutaway plan view showing the diaper as has been developed and flattened in the front-back direction after the front and rear waist regions have been peeled off at seams as viewed from the inner surface of the diaper.
{Fig. 3} Fig. 3(a) is a sectional view taken along the line III-III in Fig. 2 and Fig. 3(b) is a sectional view similar to Fig. 3(a) but illustrating the diaper put on the wearer's body.
{Fig. 4} Fig. 4 is a sectional view taken along the line IV-IV in Fig. 2.
{Fig. 5} Fig. 5 is a plan view of a crotch member.
{Fig. 6} Fig. 6 is a perspective view illustrating a part of the diaper manufacturing apparatus used to make the crotch member.
{Fig. 7} Fig. 7 is a plan view similar to Fig. 5, showing the second embodiment of the present invention.

### {Description of Embodiments}

### <First Embodiment>

Fig. 1 is a perspective view showing a disposable diaper 10 as one example of the disposable wearing articles according to a first embodiment of the present invention, Fig. 2 is a partially cutaway plan view showing the diaper 10 as has been developed and flattened in the front-back direction after front and rear waist regions have been peeled off from each other at seams 18 as viewed from the inner surface of the diaper 10, Fig. 3(a) is a sectional view taken along the line III-III in Fig. 2 and Fig. 3(b) is a sectional view similar to Fig. 3(a) but illustrating the diaper 10 put on the wearer's body, Fig 4 is a sectional view taken along the line IV-IV in Fig. 2 and Fig. 5 is a plan view of a crotch member 12. In Fig. 5, a liquid-absorbent structure 40 is not illustrated for convenience of illustration.

Referring to Figs. 1 and 2, the disposable diaper 10 has a longitudinal direction Y and a transverse direction X orthogonal to the longitudinal direction Y, an imaginary center line P-P bisecting a dimension of the diaper 10 in the transverse direction X, including a side facing the wearer's skin and a side facing away from the diaper wearer's skin, an annular elasticized waist panel 11 and a crotch member 12 attached to the side of the annular waist panel 11 to define a front waist region 13, a rear waist region 14 and a crotch region 15 extending in the longitudinal direction Y between the front and rear waist regions 13, 14. The diaper 10 is formed symmetrically about the imaginary center line P-P. The elastic waist panel 11 includes a front waist panel 16 defining the front waist region 13 and a rear waist panel 17 defining the rear waist region 14.

The front waist panel 16 has a horizontally long substantially rectangular shape contoured by an inner end 16a intersecting with the crotch member 12 and extending in the transverse direction X, an outer end 16b opposed to and spaced from the inner end 16a and extending in the transverse direction X and both side edges 16c, 16d extending in the longitudinal direction Y between the inner and outer ends 16a, 16b.

The rear waist panel 17 is substantially the same as the front waist panel 16 in size as well as in shape and its horizontally long substantially rectangular shape is contoured by an inner end 17a intersecting with the crotch member 12 and extending in the transverse direction X, an outer end 17b opposed to and spaced from the inner end 17a and extending in the transverse direction X and both side edges 17c, 17d extending in the longitudinal direction Y between the inner and outer ends 17a, 17b.

Both side edges 16c, 16d of the front waist panel 16 and both side edges 17c, 17d of the rear waist panel 17 are put flat and joined together at seams 18 arranged intermittently in the longitudinal direction Y to define a waist-opening 20 and a pair of leg-openings 21 (See Fig. 1). The seams 18 may be formed by joining means of well known art, for example, various kinds of heat-sealing techniques such as heat-embossing treatments or ultrasonic sealing treatments.

The front waist panel 16 includes a first topsheet 23 lying on the side facing the wearer's skin and a first backsheet 24 lying on the side facing away from the wearer's skin, each being formed of breathable and hydrophobic nonwoven fabrics or liquid-impervious but moisture-pervious plastic sheets or laminated sheets thereof wherein these two sheets are bonded to each other by the intermediary of hot melt adhesives (not shown) applied to at least one of these sheets 23, 24. Between the first topsheet 23 and the first backsheet 24, a plurality of strand- or string-like front waist elastic elements 25 extending in the transverse direction X are contractibly attached under tension in the transverse direction X with hot melt adhesives (not shown) so that the front waist panel 16 may be elasticized at least in the transverse direction X. It is also possible to bond the first topsheet 23 and the first backsheet 24 to each other only by the intermediary of hot melt adhesives with which the respective elastic elements constituting the front waist elastic elements 25 are coated.

The rear waist panel 17 includes a second topsheet 26 lying on the side facing the wearer's skin and a second backsheet 27 lying on the side facing away from the wearer's skin, each being formed of breathable and hydrophobic nonwoven fabrics or liquid-impervious but moisture-pervious plastic sheets or laminated sheets thereof wherein these two sheets are bonded to each other by the intermediary of hot melt adhesives (not shown) applied to at least one of these sheets 26, 27. Between the second topsheet 26 and the second backsheet 27, a plurality of strand- or string-like rear waist elastic elements 28 extending in the transverse direction X are contractibly attached under tension in the transverse direction X with hot melt adhesives (not shown) so that the rear waist panel 17 may be elasticized at least in the transverse direction X. It is also possible to bond the second topsheet 26 and the second backsheet 27 only by the intermediary of hot melt adhesive with which the respective elastic elements constituting the rear waist elastic elements 28 are coated.

The crotch member 12 is contoured by a front end 31 extending in the transverse direction X in the front waist region 13, a rear end 32 opposed to and spaced from the front end 31 in the longitudinal direction and extending in the transverse direction X in the rear waist region 14 and both side edges 33, 34 spaced from and opposed to each other in the transverse direction X and extending in the longitudinal direction Y. In the diaper 10 having been developed and flattened, the crotch member 12 is substantially formed of a vertically long rectangle (See Fig. 2).

The crotch member 12 has a front end 35 attached to the outer surface of the front waist panel 16, a rear end 36 attached to the outer surface of the rear waist panel 17, an intermediate zone 37 extending in the longitudinal direction Y between the front and rear ends 35, 36, a crotch topsheet 38 formed of a plastic film, a crotch backsheet 39 formed of a breathable and hydrophobic nonwoven fabric and a liquid-absorbent structure 40 attached to the side of the crotch topsheet 38 facing the wearer's skin. The crotch topsheet 38 and the crotch backsheet 39 are bonded to each other with hot melt adhesives (not shown) applied to at least one of these two sheets 38, 39. Both lateral portions of these two sheets 38, 39 bonded to each other are folded inward to define a pair of elasticized lateral portions 41 extending in the longitudinal direction Y on the inner surface of the liquid-absorbent structure 40. The liquid-absorbent structure 40 at least includes a semi-rigid absorbent core formed from a mixture of super-absorbent polymer particles and fluff pulp fibers having stiffness higher than the other sheets and wrapped with a liquid-pervious sheet.

The respective elasticized lateral portions 41 have inner side edges 42 defined by the respective side edges of the crotch top- and backsheets 38, 39 bonded together, front and rear ends 44, 45 and outer side edges 43 defined by fold lines (corresponding to the both side edges 33, 34 of the crotch member 12) along which the elasticized lateral portions 41 are folded inward in the course of making the diaper 10. At the front and rear ends 44, 45 of the respective elasticized lateral portions 41, front and rear ends of the crotch topsheet 38 defining the respective elasticized lateral portions 41 and front and rear ends of the crotch backsheet 39 are sealed together with adhesive regions 48, 49 extending in the transverse direction X, respectively. In consequence, body waste flowing and permeating into the respective elasticized lateral portions 41 through the front and rear ends 44, 45 should not leak out of the diaper 10. Though not illustrated, the front and rear ends 44, 45 of the elasticized lateral portions 41 are secured to the crotch topsheet 38 defining the front and rear ends 31, 32 of the crotch member 12 by various types of bonding means such as adhesives. With the front and rear ends 44, 45 of the elasticized lateral portions 41 secured to the front and rear ends 31, 32 of the crotch member 12 in this manner, body waste excreted onto the liquid-absorbent structure 40 should not leak out of the diaper 10 through a gap which would otherwise be left between the front and rear ends 44, 45 of the elasticized lateral portions 41 and the front and rear ends 31, 32 of the crotch member 12. The adhesive regions 48, 49 may be formed by various types of the same adhesives as or different from the hot melt adhesives applied to the entire inner surfaces of the crotch top- and backsheets 38, 39, or the other means such as heat sealing treatments, and ultrasonic sealing treatments or combinations thereof.

Referring to Figs. 2 through 5, the elasticized lateral portions 41 are respectively provided with a plurality of strand- or string-like first leg elastic elements 50 and a plurality of strand- or string-like second leg elastic elements 51 and thereby the elasticized lateral portions 41 are elasticized at least in the longitudinal direction Y. The first and second leg elastic elements 50, 51 are sandwiched between the two sheets 38, 39 and secured thereto under tension in the longitudinal direction Y with hot melt adhesives (not shown). Specifically, in the disposable diaper for adult, front and rear ends 50a, 51a, 50b, 51b of the first and second leg elastic elements 50, 51 are located at a distance of about 15 to about 100mm from the front and rear ends 44, 45 of the elasticized lateral portions 41. While the first and second leg elastic elements 50, 51 are provided in the form of strand- or string-like elastic elements according to the present embodiment, sheet-like elastic elements may be used so long as the desired effect of the present invention can be obtained.

The first and second leg elastic elements 50, 51 have the front and rear ends 50a, 51a, 50b, 51b are retracted inward from the front and rear ends 44, 45 by so-called cut-back and, for the purpose of uplift prevention, preferably these front and rear ends 50a, 51a, 50b, 501b are locally coated with larger amount of adhesives than the amount of adhesives applied to the other regions. More specifically, the basis mass of adhesives to fix the first and second leg elastic elements 50, 50 as whole between the crotch top- and backsheets 38, 39 is preferably in a range of 0.5 to 10.0g/m² while the basis mass of adhesives to sandwich the front and rear ends 50a, 51a, 50b, 51b between the two sheets 38, 39 is preferably in a range of 3 to 80g/m².

As illustrated in Fig. 3(a) and Fig. 4, crotch top- and backsheets 38, 39 having respective inner ends integrally sealed together. Specifically, these sheets 38, 39 are integrate with hot melt adhesives applied thereto in a planar or fibrous pattern to assure sealed condition within the respective elasticized lateral portions 41 and thereby to prevent the first leg elastic elements 50 from falling off through the inner side edges 42 of the respective first leg elastic elements 50.

Referring to Fig. 3(b), an apparent dimension of each of the elasticized lateral portions 41 in the transverse direction X is defined by the dimension between the inner side edge 42 and the outer side edge 43 as long as the elasticized lateral 41 is kept in an inwardly folded state. However, under the effect of the first and second leg elastic elements 50, 50 secured to the respective elasticized lateral portions 41, these elasticized lateral portions 41 raise themselves toward the wearer's body and the crotch region 15 is sterically deformed. In consequence, the elasticized lateral portions are spaced upward from the liquid-absorbent structure 40 and come in contact with the wearer's inguinal regions. With the diaper 10 put on the wearer's body, the crotch member 12 has a shape like a bag suspended from the elasticized waist panel 11 (See Fig. 1). When body waste is excreted onto the liquid-absorbent structure 40 in such a state of the diaper 10 put on the wearer's body, the liquid-absorbent structure 40 is spaced downward from the wearer's buttocks under its own weight and thereby a body waste retention space for temporary retention of body waste is formed between the wearer's body and the liquid-absorbent structure 40.

The first leg elastic elements 50 include three (3) elastic elements rectilinearly extending along the inner side edge 42 of the elasticized lateral 41 in the longitudinal direction Y. The second leg elastic elements 51 include a plurality of elastic elements each including concavely curved segment 53 in the middle zone of the crotch region 12 and rectilinear segments 54, 55 being contiguous to the concavely curved segment 53 and extending in the vicinity of the front and rear ends 35, 36 of the crotch member 12.

More specifically, a width dimension L₁ of each of the elasticized lateral portions 41 in the transverse direction X is preferably in a range of 30 to 120mm, more preferably, in a range of 60 to 100mm and a dimension by which the first and second leg elastic elements 50, 50 are spaced from each other in the transverse direction X is in a range of 3 to 20mm. A dimension L₂ by which the outermost point 53a of the concavely curved segment 53 is spaced from the outer side edge 43 of the elasticized lateral 41 is in a range of about 20 to about 50mm. In this way, the first and second leg elastic elements 50, 50 get closer to each other in the middle zone of the crotch region 15 and the elastic elements are locally gathered to enhance a tensile stress. Consequently, it is possible to put these zones in close contact with the wearer's skin and thereby to prevent body waste from sideways leaking in an effective manner. In addition, the tensile stress can be locally enhanced without adjusting the tensile stress of the elastic elements themselves and/or partially increasing the number of the elastic elements. This leads to reduction of cost and simplification of a manufacturing process. In the vicinity of the ends of the respective elasticized lateral portions 41, the first leg elastic elements 50 are spaced from the second leg elastic elements 51 by a given distance so that these ends may come in planar contact with the wearer's skin and thereby prevent body waste from leaking out in the vicinity of these ends.

Referring to Fig. 3(b), the respective elasticized lateral portions 41 have the required width dimension L₁ in the transverse direction X and the concavely curved segments of the second leg elastic elements 51 are spaced from the side edges of the liquid-absorbent structure 40 by the required dimension in the middle zone of the crotch region 15 and in the vicinity thereof. With the diaper 10 of this arrangement put on the wearer's body, the regions provided with the first and second leg elastic elements 50, 51 are held in contact with the wearer' s inguinal regions and the regions defined between the respective outer side edges 53a of the second leg elastic elements 51 and the associated side edges of the liquid-absorbent structure 40, inclusive of the regions each having the width dimension L₂ trail down. Compared to the case in which the second leg elastic elements 51 rectilinearly extend in the longitudinal direction Y and/or the elasticized lateral portions 41 have an insufficient height dimension, the present embodiment makes it possible to form a relatively large space for retention of body waste and thereby to protect the wearer' s skin from being soiled with body waste. If the height dimension of the elasticized lateral portions 41 is 30mm or less, it is impossible to form the body waste retention space of the desired volume and there is possibility that the wearer' s skin might be soiled with body waste. If the height dimension of the elasticized lateral portions 41 exceeds 120mm, the volume of the body waste retention space will become too large to follow movement of the wearer's body and the inner side edges 42 of the respective elasticized lateral portions 41 will get close to or overlap with each other. As a result, the opening defined between the inner side edges 42 will become narrower and, in some cases, there is a possibility that body waste could not properly excreted onto the liquid-absorbent structure 40.

The length dimension L₂ as has been described above is preferably set in a range of 0.20 to 0.95 times and more preferably set in a range of 0.30 to 0.85 times of the width dimension L₁. If the length dimension L₂ is less than 0.20 times of the width dimension L₁, the dimension by which the first leg elastic elements 50 are spaced from the concavely curved segments 53 of the second leg elastic elements 51 will be unacceptably widened and the elastic elements may not be locally gathered. In consequence, the desired tensile stress may not be created and eventually the effect of the present invention may not be obtained. If the length dimension L₂ exceeds 0.95 times of the width dimension L₁, the concavely curved segments 53 of the second leg elastic elements 51 may intersect with the first leg elastic elements 50 depending on various factors such as the number and the location of the first leg elastic elements 50 and an excessively high tensile stress may be generated, with apprehensive result such that the wearer should experience uncomfortable feeling and pressure marks left on the his or her skin. It should be noted here that, while the elasticized lateral portions 41 of the crotch region 15 are provided with the first and second leg elastic elements 50, 51 according to the present embodiment, these leg elastic elements 50, 51 may be arranged not on the elasticized lateral portions 41 but directly on the side edges of the crotch region 15 so long as the same effect as that of the present invention can be obtained.

The tensile stress in the elasticized regions defined by the first leg elastic elements 50 is preferably higher than that in the elasticized regions defined by the second leg elastic elements 51. Specifically, at the time point of 80% stretch, the tensile stress in the elasticized regions defined by the first leg elastic elements 50 is about 1.05N/10mm and the tensile stress in the elasticized regions defined by the second leg elastic elements 51 is about 0.49N/10mm according to the present embodiment. By setting the tensile stress in the regions closest to the wearer's inguinal regions, i.e., in the elasticized regions defined by the first leg elastic elements 50 to be higher than the tensile stress in the elasticized regions in the vicinity of the inner side edges 41 of the elasticized lateral portions 41, possibly occurring sideways leakage of body waste can be further effectively prevented. In addition, the dimension by which the inner side edges of the respective elasticized lateral portions 41 are spaced from each other can be sufficiently assured and these elasticized lateral portions 41 can raise themselves sufficiently. In this way, the volume of the body waste retention space formed above the crotch member 12 can be further enlarged. It should be appreciated here that the respective tensile stress of the first and second leg elastic elements 50, 51 may be selectively set by varying various factors such as the number and the stretch ratio of the elastic elements.

The tensile stress in the regions elasticized by the first and second leg elastic elements 50, 51 is measured by the method as described below.

The diaper 10 is developed to put the first and second leg elements 50, 51 in a stretched state. A portion of the elasticized lateral 41 including the first leg elastic elements 50 is cut off as a test piece 1 and a portion of the elasticized lateral 41 including the rectilinear segment 54, 55 of the second leg elastic elements 51 is cut off as a test piece 2. Each of the first and second test pieces is a strip having a dimension (including the inner- and outermost elastic elements) of about 10mm in the transverse direction X of the diaper 10 and a dimension of about 160mm (including the length of 30mm overlapped by the chucks of the tester) in the longitudinal direction Y of the diaper 10. The method is based on measurement of region width (a dimension in the longitudinal direction Y of the diaper 10) of the respective test pieces. Each of the test piece 1 and the test piece 2 is secured in a contracted state between chucks (the initial chuck distance is set to a range of 20 to 30mm and appropriately adjusted if desired) of the Tensile Tester (manufactured by Shimadzu Corporation in Japan). From the initial state, the test piece is stretched at a tension rate of 100mm/min in the direction corresponding to the longitudinal direction Y of the diaper 10 and a load (mN) is measured at the moment the test piece has been stretched to 80% of the maximally stretched length (80% of the maximally stretched length of the test piece 1 or 2 corresponding to the maximum displacement in the longitudinal direction Y of the diaper 10). Tensile stress is calculated from a calculating formula of measured value (mN) / region width (mm).

Referring again to Figs. 2 - 4, the crotch member 12 is attached to the respective outer surfaces of the front and rear waist panels 16, 17 in a front adhesive region 61 and a rear adhesive region 62 defined by hot melt adhesives applied to the surface of the front end 35 and the rear end 36 of the crotch member 12 facing the wearer's skin, respectively. The front adhesive region 61 substantially overlaps the inner end 16a of the front waist panel 16 and has a substantially trapezoidal shape contoured by an inner end 61a extending in the transverse direction X between the inner side edges 42 of the respective elasticized lateral portions 41 of the crotch member 12, an outer end 61b substantially overlapping the front end 31 of the crotch member 12 and extending in the transverse direction X and both side edges 61c extending between the inner and outer ends 61a, 61b obliquely with respect to the center line P-P so that each of these side edges 61c extends outward as viewed in the transverse direction X as it extends outward as viewed in the longitudinal direction Y. The rear adhesive region 62 has a substantially rectangular shape which is horizontally long and includes an inner end 62a substantially overlapping the inner end 17a of the rear waist panel 17 and extending in the transverse direction X and an outer end 62b substantially overlapping the rear end 32 of the crotch member 12 and extending in the transverse direction X. In the front and rear adhesive regions 61, 62, sub-regions 63 (corresponding to shaded portions in Fig. 2) in which the inner surface of the liquid-absorbent structure 40 is bonded to the respective outer surfaces of the front and rear waist panels 16, 17 may be left not coated with adhesive. In this case, void spaces are defined between the liquid-absorbent structure 40 and the front and rear waist panels 16, 17 so that these void spaces may function as pockets adapted to absorb and to retain body waste.

By attaching the front and rear ends 35, 36 to the respective outer surfaces of the front and rear waist panels 16, 17, the volume of the body waste retention space can further enlarged. It should be appreciated that one of the front end 35 and the rear end 36 may be attached to the outer surface of the associated one of the waist panels 16, 17 so long as the volume of the body waste retention space can be effectively enlarged. When the rear end 36 is attached to the outer surface of the rear waist panel 17, the body waste retention space can be shaped in accordance with the shape of the wearer's buttocks and thereby feeling to wear the diaper 10 can be improved.

Fig. 6 is a perspective diagram illustrating a part of an apparatus for making the diaper 10, more particularly, illustrating a step of making the crotch member 12. In Fig. 6, the machine direction in which first and second webs 138, 139 are fed is designated by MD and the direction intersecting with this is designated by CD.

Referring to Fig. 6, first web 138 as stock materials for the crotch topsheet 38 is stretched by a tension roll (not shown) extending in the cross direction CD and then guided by a guide roll 140 to a press step 143 including a pair of press rolls 141, 142. Second web 139 as stock materials for the crotch backsheet 39 is stretched by a tension roll (not shown) extending in the cross direction CD and then fed to an adhesive coating step 144. In the adhesive coating step 144, the entire inner surface 139a of the second web 139 is coated with hot melt adhesives to bond the first and second webs 138, 139 to each other and simultaneously to sandwich a pair of first continuous elastic members 150 as stock material for the first leg elastic elements 50 and a pair of second continuous elastic members 151 as stock material for the second leg elastic elements 51 between the two webs 138, 139 and to secure them therebetween. After having been coated with hot melt adhesive, the second web 139 is guided to a press step 143.

In the press step 143, the first continuous elastic members 150 are arranged to be rectilinear in the machine direction MD while the second continuous elastic members 151 are oscillated at high velocity by oscillating arms 146 in the cross direction CD so as to draw undulation repeated in the cross direction CD. The first and second continuous elastic members 150, 151 are pressed together with the first web 138 and the second web 139 to form composite web 160. Lateral portions of composite web 160 extending in the machine direction MD and opposed to each other in the cross direction CD are folded inward to form the elasticized lateral portions 41.

In the composite web 160 formed in this manner, concavely curved segments of the second continuous elastic members 151 (corresponding to the concavely curved segments 53 in the second leg elastic elements 51) are pulled in the cross direction CD with a force stronger than a force exerted on the rectilinear segments 162 extending in front of and behind (corresponding to the rectilinear segments 54, 55 of the first leg elastic elements 51) and thereby generate a tensile stress higher than that generated in the rectilinear segments 162. As a result, in the rectilinear segments 162, the second continuous elastic members 151 are slightly stretched or not stretched at all in the cross direction CD. Consequentially, in the respective elasticized lateral portions 41, the concavely curved segments 53 of the second leg elastic elements 51 come in close contact with the wearer's inguinal regions and contribute to improvement in fit and, at the same time, allow the body waste retention space having a large volume to be formed. Relatively low tensile stress generated in the rectilinear segments 54, 55 serves to prevent the workability from lowering due to interference between the tensile stress when the front and rear ends 35, 36 of the crotch member 12 are attached to the elasticized waist panel 11. It is also possible to set the tensile stress of the concavely curved segments 161 to be lower than that of the rectilinear segments 162 by, for example, regulating the oscillation amplitude of the oscillating arms 146.

### <Second Embodiment>

Fig. 7 is a plan view of the crotch member 12 similar to Fig. 5, illustrating a second embodiment of the present invention. For convenience of illustration, like Fig. 5, the liquid-absorbent structure 40 is indicated by imaginary lines and basic features of the diaper 10 are similar to those in the first embodiment. Only dissimilarities from the first embodiment will be described below.

Referring to Fig. 7, a dimension by which each pair of the adjacent second leg elastic elements 51 is spaced from each other in the transverse direction X is gradually reduced as these elastic elements 51 get closer to the middle zone of the crotch region 15. In other words, these adjacent elastic elements get closest to each other in the concavely curved segments 53 thereof. According to the present embodiment, the tensile stress of the elasticized lateral 41 can be set to become higher as they get closer to the middle zone of the crotch region 15 and thereby the middle zone of the crotch region 15 can be put in close contact with the wearer's skin.

As stock materials for the component members such as the elasticized waist panel 11 and the crotch member 12, various kinds of material widely used in the field of the disposable wearing article may be selectively used. While so-called pants-type diaper 10 including the elasticized waist panel 11 and the crotch member 12 separately prepared has been described as the typical example of the disposable wearing article according to the present invention, the present invention may be implemented in the form of the diaper including a substantially hourglass-shaped pair of sheets and an absorbent core sandwiched between these two sheets or in the form of so-called open-type diaper.

## Claims

1. A disposable wearing article (10) having a longitudinal direction (Y) and a transverse direction (X) orthogonal to the longitudinal direction, comprising a side facing the wearer's skin and a side facing away from the wearer's skin, a front waist region(14), a rear waist region (15) and a crotch region (15) extending between the front and rear waist regions, wherein:
the front and rear waist regions (13, 14) are defined by an annular waist panel (11), the waist panel (11) includes a front waist panel (16) defining the front waist region (13) and a rear waist panel (17) defining the rear waist region (14), and a crotch member (12) is attached to the waist panel (11),
the crotch member (12) has a front end (35) attached to the outer surface of the front waist panel (16), a rear end (36) attached to the outer surface of the rear waist panel (17), an intermediate zone (37) extending in the longitudinal direction between the front and rear ends (35, 36), a crotch topsheet (38) formed of a plastic film, a crotch backsheet (39) formed of a breathable and hydrophobic nonwoven fabric and a liquid-absorbent structure (40) attached to the side of the crotch topsheet (38) facing the wearer's skin,
lateral portions of the crotch topsheet (38) and the crotch backsheet (39) are bonded to each other with hot melt adhesives applied to at least one of these sheets (38, 39) and folded inward to define a pair of elasticized lateral portions (41) extending in the longitudinal direction on the inner surface of the liquid-absorbent structure (40),
each of the elasticized lateral portions (41) has an inner side edge (42) defined by the side edges of the crotch top- and back sheets (38, 39) bonded together, front and rear ends (44, 45) and an outer side edge (43) defined by fold lines along which the elasticized lateral portions (41) are folded inward,
the front and rear ends (44, 45) of the elasticized lateral portions (41) are secured to the front and rear ends (31, 32) of the crotch member (12) by bonding means,
the elasticized lateral portions (41) are respectively provided with a plurality of strand- or string-liise first leg elastic elements (50) and a plurality of strand- or string-like second leg elastic elements (51) and thereby the elasticized lateral portions (41) are elasticized at least in the longitudinal direction, the first and second leg elastic elements (50, 51) are sandwiched between the crotch topsheet (38) and the crotch backsheet (39) and secured thereto under tension in the longitudinal direction with hot melt adhesives,
the first leg elastic elements (50) include three elastic elements rectilinearly extending along the inner side edge (42) of the elasticized lateral portions (41) in the longitudinal direction,
the second leg elastic elements (51) extend in the longitudinal direction outboard of the first leg elastic elements (50) as viewed in the transverse direction, each of the second leg elastic elements (51) has a shape concavely curved in a longitudinally middle zone of the crotch region and in the vicinity thereof, and
elasticized regions defined by the first leg elastic elements (50) have tensile stress higher than that of elasticized regions defined by the second leg elastic elements (51).

2. The wearing article defined by Claim 1, wherein a dimension by which the second leg elastic elements (51) are spaced from the first leg elastic elements (50) is gradually reduced as the first and second leg elastic elements get closer to the middle zone of the crotch region.

3. The wearing article defined by Claim 1 or 2, wherein each of the second leg elastic elements (51) includes a concavely curved segment in the middle zone of the crotch region (15) and in the vicinity thereof and a pair of rectilinear segments being contiguous to the concavely curved segment and respectively extending to ends of the second leg elastic elements (51) opposite in the longitudinal direction and wherein the concavely curved segment has a tensile stress higher than that of the rectilinear segment.

## Patentansprüche

1. Wegwerfbarer Trageartikel (10) mit einer Längsrichtung (Y) und einer zu der Längsrichtung orthogonalen Querrichtung (X), umfassend einer der Haut des Trägers zugewandten Seite und einer der Haut des Trägers abgewandten Seite, einem vorderen Taillenbereich (14), einem hinteren Taillenbereich (15) und einem Schrittbereich (15), der sich zwischen dem vorderen und dem hinteren Taillenbereich erstreckt, wobei:
der vordere und hintere Taillenbereich (13, 14) von einer ringförmigen Taillenpaneele (11) bestimmt werden, wobei die Taillenpaneele (11) ein den vordere Taillenbereich (13) bestimmendes vorderes Taillenpaneel (16) und ein den hinteren Taillenbereich (14) bestimmendes hinteres Taillenpaneel (17) umfasst und wobei ein Schrittelement (12) an dem Taillenpaneel (11) befestigt ist,
das Schrittelement (12) ein vorderes Ende (35), das an der äußeren Oberfläche der vorderen Taillenpaneele (16) befestigt ist, ein hinteres Ende (36), das an der äußeren Oberfläche der hinteren Taillenpaneele (17) befestigt ist, eine dazwischenliegende Zone (37), die sich in Längsrichtung zwischen den vorderen und hinteren Enden (35, 36) erstreckt, eine Schrittoberlage (38), die aus einer Kunststoffolie gebildet ist, und eine Schrittunterlage (39) hat, die aus einem atmungsaktiven und wasserabweisenden Vliesstoff und einer an der Seite der Schrittoberlage (38), die der Haut des Trägers zugewandt ist, befestigte flüssigkeitsabsorbierenden Struktur (40) gebildet ist,
seitliche Abschnitte der Schrittoberlage (38) und der Schrittunterlage (39) miteinander mit Heißschmelzkleber, der auf zumindest einer dieser Lagen (38, 39) aufgebracht ist, verklebt und nach innen gefaltet sind, um ein Paar von elastischen seitlichen Abschnitten (41) zu bestimmen, das sich in die Längsrichtung auf der inneren Oberfläche der flüssigkeitsabsorbierenden Struktur (40) erstreckt,
jeder der elastischen seitlichen Abschnitte (41) eine innere Seitenkante (42) hat, die durch die Seitenkanten der zusammengeklebten Schrittober- und Unterlage (38, 39), vordere und hintere Enden (44, 45) und eine durch Faltlinien, entlang derer die elastischen seitlichen Abschnitte (41) nach innen gefaltet sind, bestimmte äußere Seitenkante (43) bestimmt ist,
die vorderen und hinteren Enden (44, 45) der elastischen seitlichen Abschnitte (41) an die vorderen und hinteren Enden (31, 32) des Schrittelements (12) durch Klebemittel befestigt sind,
die elastischen seitlichen Abschnitte (41) jeweils mit einer Vielzahl von strang- oder fadenartigen ersten Beinelastikelementen (50) und einer Vielzahl von strang- oder fadenartigen zweiten Beinelastikelementen (51) versehen sind und dadurch die elastischen seitlichen Abschnitte (41) zumindest in die Längsrichtung elastisch sind, die ersten und zweiten Beinelastikelemente (50, 51) zwischen der Schrittoberlage (38) und der Schrittunterlage (39) sandwichartig angeordnet sind und daran unter Spannung in die Längsrichtung mit Heißschmelzkleber befestigt sind,
die ersten Beinelastikelemente (50) drei Elastikelemente umfassen, die sich geradlinig entlang der inneren Seitenkante (42) der elastischen seitlichen Abschnitte (41) in die Längsrichtung erstrecken,
sich die zweiten Beinelastikelemente (51) in die Längsrichtung, in die Querrichtung gesehen außerhalb der ersten Beinelastikelemente (50) erstrecken, wobei jedes der zweiten Beinelastikelemente (51) in einer länglichen Mittelzone des Schrittbereichs und in der Umgebung davon eine konkav gekrümmte Form hat, und
elastische Bereiche, die durch die ersten Beinelastikelemente (50) bestimmt sind, eine höhere Zugspannung haben als die von elastischen Bereichen, die von den zweiten Beinelastikelementen (51) bestimmt werden.

2. Trageartikel nach Anspruch 1, wobei eine Abmessung, um die die zweiten Beinelastikelemente (51) von den ersten Beinelastikelementen (50) beabstandet sind, mit dem Annähern des ersten und zweiten Beinelastikelements an die Mittelzone des Schrittbereichs allmählich reduziert wird.

3. Trageartikel nach Anspruch 1 oder 2, wobei jedes der zweiten Beinelastikelemente (51) ein konkav gekrümmtes Segment in der Mittelzone des Schrittbereichs (15) und in der Umgebung davon und ein Paar von geradlinigen Segmenten umfasst, die zu den konkav gekrümmten Segmenten angrenzen und sich jeweils zu den in die Längsrichtung gegenüberliegenden Enden der zweiten Beinelastikelementen (51) erstrecken und wobei die konkav gekrümmten Segmente eine Zugspannung aufweisen, die höher ist als die des geradlinigen Segments.

## Revendications

1. Article à porter jetable (10) présentant une direction longitudinale (Y) et une direction transversale (X) perpendiculaire à la direction longitudinale, comprenant une face tournée vers la peau du porteur de l'article et une face opposée à la peau du porteur de l'article, une région de taille avant (14), une région de taille arrière (15) et une région d'entrejambe (15) s'étendant entre les régions de taille avant et arrière, dans lequel :
les régions de taille avant et arrière (13, 14) sont définies par un panneau de taille annulaire (11), le panneau de taille (11) comporte un panneau de taille avant (16) définissant la région de taille avant (13) et un panneau de taille arrière (17) définissant la région de taille arrière (14), et un membre d'entrejambe (12) est attaché au panneau de taille (11),
le membre d'entrejambe (12) présente une extrémité avant (35) attachée à la surface extérieure du panneau de taille avant (16), une extrémité arrière (36) attachée à la surface extérieure du panneau de taille arrière (17), une zone intermédiaire (37) s'étendant dans la direction longitudinale entre les extrémités avant et arrière (35, 36), une feuille de dessus d'entrejambe (38) formée d'un film plastique, une feuille de dessous d'entrejambe (39) formée d'un tissu non-tissé respirable et hydrophobe et d'une structure absorbant les liquides (40) attachée à la face de la feuille de dessus d'entrejambe (38) tournée vers la peau de l'utilisateur portant l'article,
des parties latérales de la feuille de dessus d'entrejambe (38) et de la feuille de dessous d'entrejambe (39) sont reliées les unes aux autres avec des adhésifs thermofusibles appliqués sur au moins une de ces feuilles (38, 39) et sont pliées vers l'intérieur pour définir une paire de parties latérales élastiquées (41) s'étendant dans la direction longitudinale de la surface intérieure de la structure absorbant des liquides (40),
chacune des parties latérales élastiquées (41) présente un bord latéral intérieur (42) défini par les bords latéraux des feuilles de dessus et de dessous d'entrejambe (38, 39) reliées ensemble, des extrémités avant et arrière (44, 45) et un bord latéral extérieur (43) défini par des lignes de pliage le long desquelles les parties latérales élastiquées (41) sont pliées vers l'intérieur,
les extrémités avant et arrière (44, 45) des parties latérales élastiquées (41) sont biloquées au niveau des extrémités avant et arrière (31, 32) du membre d'entrejambe (12) par des moyens de liaison,
les parties latérales élastiquées (41) sont respectivement pourvues d'une pluralité de premiers éléments élastiques de jambe de type cordon ou ficelle (50) et d'une pluralité de seconds éléments élastiques de jambe de type cordon ou ficelle (51) et donc les parties latérales élastiquées (41) sont élastiquées au moins dans la direction longitudinale, les premier et second éléments élastiques de jambe (50, 51) sont pris en sandwich entre la feuille de dessus d'entrejambe (38) et la feuille de dessous d'entrejambe (39) et sont bloqués au niveau de ces dernières sous tension dans la direction longitudinale à l'aide d'adhésifs thermofusibles,
les premiers éléments élastiques de jambe (50) comportent trois éléments élastiques s'étendant de manière rectiligne le long du bord latéral intérieur (42) des parties latérales élastiquées (41) dans la direction longitudinale,
les seconds éléments élastiques de jambe (51) s'étendent dans la direction longitudinale à l'extérieur des premiers éléments élastiques de jambe (50) vus dans la direction transversale, chacun des seconds éléments élastiques de jambe (51) présente une forme incurvée de manière concave dans une zone centrale longitudinalement de la région d'entrejambe et à proximité de cette dernière, et
des régions élastiquées définies par les premiers éléments élastiques de jambe (50) présentent une tension de traction supérieure à celle des régions élastiquées définies par les seconds éléments élastiques de jambe (51).

2. Article à porter selon la revendication 1, dans lequel une dimension, par laquelle les seconds éléments élastiques de jambe (51) sont espacés des premiers éléments élastiques de jambe (50), est progressivement réduite au fur et à mesure que les premier et second éléments élastiques de jambe s'approchent de la zone centrale de la région d'entrejambe.

3. Article à porter selon la revendication 1 ou 2, dans lequel chacun des seconds éléments élastiques de jambe (51) comporte un segment incurvé de manière concave dans la zone centrale de la région d'entrejambe (15) et à proximité de cette dernière et une paire de segments rectilignes contigus au segment incurvé de manière concave et s'étendant respectivement vers des extrémités des seconds éléments élastiques de jambe (51) à l'opposé dans la direction longitudinale et dans lequel le segment incurvé de manière concave présente une tension de traction supérieure à celle du segment rectiligne.
